# EUROPEAN PATENT SPECIFICATION

(11) **EP 0 855 224 B1**
(45) Date of publication and mention of the grant of the patent: **06.05.2004**
(21) Application number: 98300547.1
(22) Date of filing: 27.01.1998
(51) Int. Cl.: B05B 7/00, A61M 11/06

(54) **Atomizer**
Zerstäuber
Atomiseur

(30) Priority: 27.01.1997 GB 9701621
(43) Date of publication of application: 29.07.1998
(73) Proprietor: Profile Respiratory Systems Limited, Bognor Regis, West Sussex PO22 9SL (GB)
(72) Inventor: Pitcher, Jay, Westmead, New South Wales 2145 (AU); Le Cheminant, David, Newtown, New South Wales 2042 (AU)
(74) Representative: Wright, Howard Hugh Burnby

(56) References cited:
- FR-A- 2 751 883
- US-A- 2 535 844
- US-A- 3 603 308
- US-A- 5 570 682
- US-A- 5 823 179

## Description

This invention relates to atomizers, in particular, atomizers for dispensing powders or liquids which are atomised by a stream of compressed air. Such atomizers may, for example, be used as medical atomizers for dispensing small quantities of medicaments.

An atomizer according to preamble of claim 1 is known from US-A-2 535 844.

Most conventional atomizers of the above type operate continuously whether atomization is required or not. Strictly speaking, when such atomizers, frequently called nebulisers, are used in medical applications, atomization is only required during the inhalation phase of a breathing cycle so that a drug can be administered by 'deposition in the lungs. In practice a patient usually inhales for about 30 percent of the breathing cycle, consequently, use of a continuously operating atomizer results in a large proportion of the atomised drug being wasted.

Some designs of medical atomizer overcome such wastage by giving the patient a manual trigger to start the atomization when they begin to inhale. Such a manual trigger controlled type of atomizer is not satisfactory since the patient must coordinate inhalation with trigger operation.

In one conventional atomizer a gas duct leads gas under pressure to a gas exit, a reservoir for holding the substance to be atomised is formed around the base of the gas duct, and a sleeve placed around the gas duct defines a passageway through which the substance to be atomised may pass to at least one outlet. A fixed deflector in the form of a bar is disposed in line with the gas outlet so that gas issuing from the gas exit is deflected so as to pass over the outlet or outlets. The passage of gas over each outlet draws the substance to each outlet. The deflected gas atomises the substance, and atomised particles of the substance are carried away by the stream of deflected gas, and are subsequently inhaled during the inhalation phase of the patient. Since the patient breathes air or gas in through the atomizer, but only inhales through part of the cycle, some of the drug is lost while the patient is not inhaling.

An example of such an atomiser system is to be found in EP-A-627,266 (Medic-Aid Limited).

According to the present invention, an atomiser comprises an atomiser jet, the atomiser jet having a product outlet from which product can be atomised, means for sourcing compressed air, a product reservoir, means of supplying the product from the reservoir to the outlet, a first chamber into which atomised product can diffuse, and means to prevent or stop the product from being atomised characterised in that the means to prevent or stop atomisation is configured to allow positive air pressure within the atomiser to be fed back into the means for supplying the product between the reservoir and the product outlet to stop the passage of the product to the product outlet in response to a positive pressure in the atomiser.

In embodiments of the invention, it is preferred that the apparatus is one which generates atomized product when the pressure in the apparatus is atmospheric or less than atmospheric (i.e. negative), but that atomisation is prevented or stopped when the pressure in the apparatus is positive (i.e. greater than atmospheric).

Further preferred and advantageous features are defined in claims 2 to 15.

In embodiments of the invention, the nebulizer may dose the medicament to the user via a mouthpiece, though it is envisaged that atomized medicament could be dosed to the user through a sealing face mask.

The invention is further described by way of example only, with reference to the accompanying drawings, in which:
Figure 1 shows a schematic cross-section of an embodiment of the invention in use with the user inhaling;
Figure 2 shows a schematic cross-section of the embodiment of Figure 1 in use with the user exhaling; and
Figure 3 shows an enlarged schematic cross section view of a secondary chamber in the embodiment of Figure 1.

Referring to the figures, the atomizer includes a gas duct 6 which provides gas under pressure (show as a series of arrowheads in gas duct 6) from a compressed air source to a gas exit 4 within a jet head 3. The gas duct 6 passes through a wall of a reservoir 7 within which a substance to be atomised such as a medicament is held. A sleeve 8 is disposed around the jet head 3 and the gas duct 6. Passages are formed between the inner surface of the sleeve 8 and the outer surface of the gas duct 6 for leading the substance to be atomised from the reservoir 7 to a secondary reservoir 1, which has an exit to outlet 5 in the jet head adjacent to the gas exit 4.

For atomization of the substance to take place, gas exiting from the gas exit 4 passes close to the outlet 5. In a manner well known in the art, gas exiting from gas exit 4 flows at high speed into baffle bar 20, which is located generally at the bottom of a first chamber 9, and it is this collision of gas which generates the appropriate conditions in which to cause atomisation of medicament. The atomisation of medicament at outlet 5 causes substance to be atomised to be drawn from the reservoir 7, through the passage between the sleeve 8 and the gas duct 6, and up through secondary reservoir 1 to the outlet 5. The flow of pressurised air when it has rebounded off of baffle 20 atomises the substance as the substance leaves the outlet 5.

Further information on the atomisation procedure may be found in EP-A-627,266.

It is important that the substance is atomised into very fine droplets. In medical applications, the substance to be atomised is a drug for administering to a patient by lung deposition. The finer the droplets, the deeper into the lungs the drug will pass. This maximises the deposition of the drug.

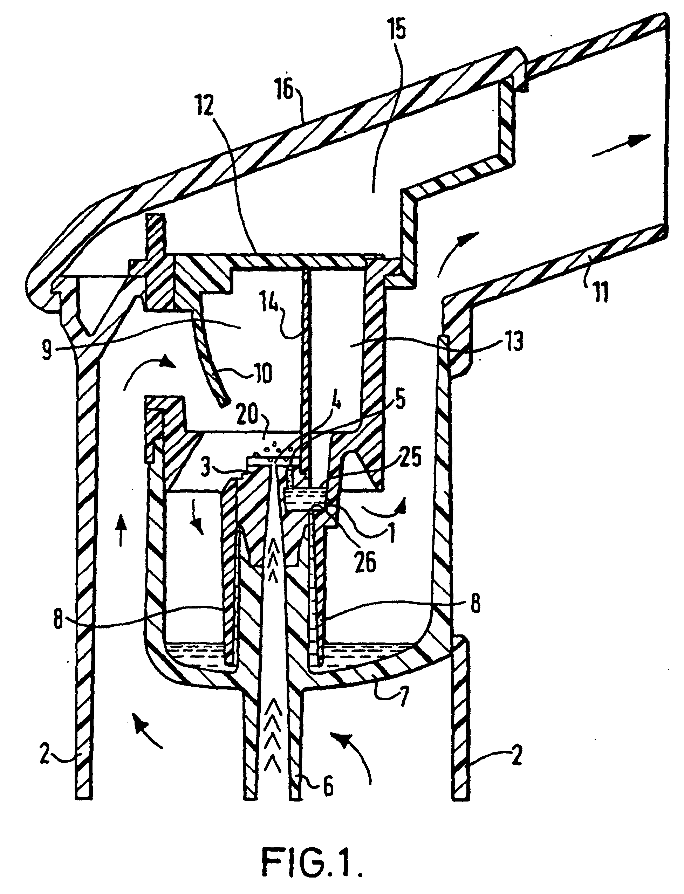

The atomizer also has a housing portion 2 which vents to be outside by which atmospheric air may enter the atomizer. However, mounted on top of gas duct 6, and integral therewith is first chamber 9 which opens towards housing portion 2, but is separated therefrom by a first one way valve 10. First one way valve 10 is resiliently deformable, and configured so as to only open and allow air from housing portion 2 into the atomizer when the user is inhaling, and hence when a reduced or negative pressure is created in first chamber 9. This is the configuration of the device shown in Figure 1.

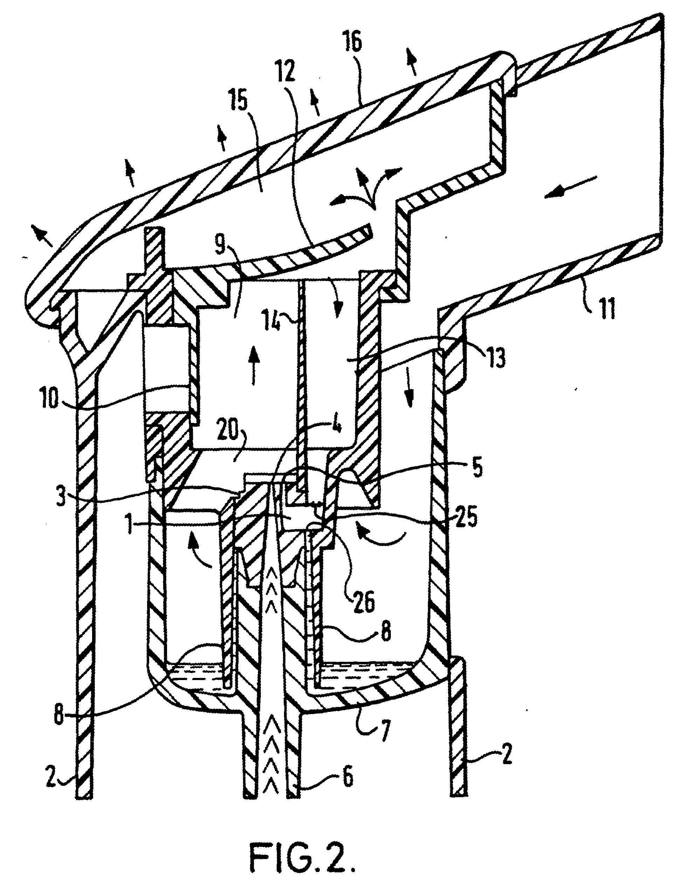

The atomizer is provided with a mouthpiece 11, which is in communication with a head space above reservoir 7, and with the first chamber 9, through which the user inhales and exhales. First chamber 9 has also located at its end remote from the gas exit 4 a second one way valve 12, which is configured to open only when the user is exhaling, and hence there is a positive pressure in first chamber 9, as shown in Figure 2.

First chamber 9 is separated from a second chamber 13 by a rigid sidewall 14, with sidewall 14 in one direction extending down towards and connecting with jet head 3, and in the other direction being also closed by second one way valve 12. Second chamber 13 is generally circular in cross section and bounded by. sidewall 14, and is configured such that it also has an exit into secondary reservoir 1. Second one way valve 12 is made of a resilient material, and is configured so as to close both first chamber 9 and second chamber 13 simultaneously when there are no positive or reduced pressures in the atomizer.

In order that the atomizer is configured so as to function smoothly, it has been found preferable that the area of second one way valve 12 covering the first chamber 9 is substantially greater than the area of the second one way valve 12 covering the second chamber 13, and preferably is greater by a ratio of at least 10:1. If this is not the case, in the exhalation phase of use the pressure on the second one way valve may be too high to allow it to open properly.

Located on the side of the second one way valve 12 is a space 15, which is bounded by a resilient filter 16. Filter 16 is air permeable, though it could also be airtight in various embodiments.

It has also been found desirable in constructing the atomizer according to the invention that the entrance to the second reservoir 1 from the second chamber 13 and adjacent the end of sidewall 14 (marked as entrance 25 in the figures) has a relatively large diameter, and is preferably at least 5 mm across (though it can have any convenient cross section shape). This is to prevent liquid bridging across this area, for example, under the effect of surface tension. It is also preferred that at the entrance of the passages between sleeve 8 and gas duct 6 to secondary reservoir 1 (marked as entrance 26 on the figures) that these are relatively small diameter, and that these passages preferably have a cross-sectional area of 1.4 mm² or less.

Such a combination of dimensions has been found to improve the cut-off of atomization of product in use which occurs when the user exhales. In particular, the relatively large entrance dimension 25 and small entrance dimension 26 facilitate the urging of medicament out of second chamber 1 and back down the passages, and hence prevent unwanted atomization.

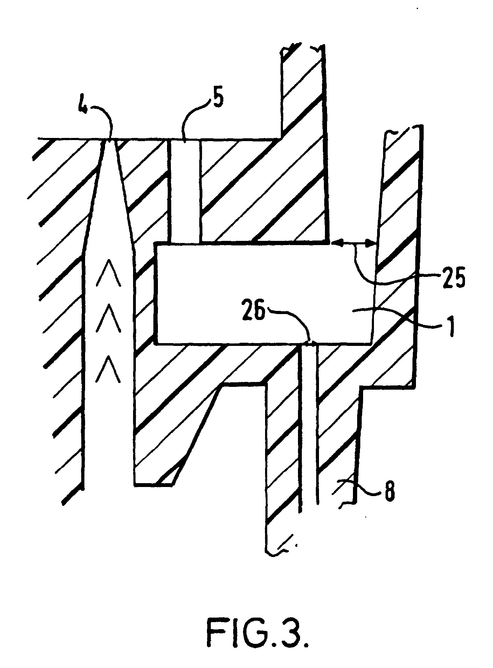

Figure 3 shows an expanded and simplified view of secondary chamber 1 showing entrances 25 and 26 in more detail.

In use, the atomizer according to the invention is connected to a compressed air source, which introduces a positive pressure through gas duct 6 into the atomizer, and through gas exit 4. This causes medicament in the vicinity of outlet 5 to be atomized in a known manner. When the user inhales (as shown in figure 1), air is drawn generally out of the atomizer via the mouthpiece 11, generating a reduced pressure in the device. In particular, first one way valve 10 is caused to open, and air is drawn in from the atmosphere. As it is drawn in, it generally captures atomized medicament, which is carried in the air stream towards mouthpiece 11 and out of the apparatus.

However, when the user has finished inhaling and subsequently exhales, this causes a positive pressure to be introduced into the atomizer, as is represented in figure 2. In particular, first one way valve 10 is sealed closed, but second one way valve 12 is caused to open. This in turn generates a positive pressure in second chamber 13, and this positive pressure causes expulsion of medicament from secondary reservoir 1, driving medicament back from outlet one down the channels in sleeve 8.

The effect of this is that as no medicament is to be found at this stage in the secondary reservoir 1 or outlet 5, no medicament is atomized during the exhaling part of the dosage cycle. This results in a saving in terms of the medicament used, since it is not atomized in the exhalation part of the cycle, and hence is not lost anywhere.

Any resultant extra positive pressure in the atomizer may be dissipated through resilient filter 16, which is air permeable.

## Claims

1. An atomiser comprising an atomiser jet, the atomiser jet having a product outlet (5) from which product can be atomised, means for sourcing compressed air, a product reservoir (7), means (6,8) of supplying the product from the reservoir (7) to the outlet (5), a first chamber (9) into which atomised product can diffuse, and means to prevent or stop the product from being atomised, **characterised in that** the means to prevent or stop atomisation is configured to allow positive air pressure within the atomiser to be fed back into the means (6,8) for supplying the product between the reservoir (7) and the product outlet (5) to stop the passage of the product to the product outlet (5) in response to a positive pressure in the atomiser.

2. An atomiser according to claim 1, **characterised in that** the means to prevent or stop atomisation includes a secondary reservoir (1) through which the product passes before reaching the product outlet (5) and which is in fluid connection with a second chamber (13), the second chamber (13) being operable in response to a positive pressure in the atomiser to drive fluid from the secondary reservoir (1).

3. An atomiser according to claim 2, **characterised in that** the second chamber (13) includes an opening, and **in that** the atomiser further comprises a one-way valve (12) which closes the opening in the second chamber (13).

4. An atomiser according to claim 3, **characterised in that** the one-way valve (12) is movable between a position closing the opening in the second chamber (13) which position permits the product to pass from the reservoir (7) to the outlet (5) and an open position which prevents or stops the product from passing from the reservoir (7) to the product outlet (5).

5. An atomiser according to claim 3 or 4, **characterised by** means for controlling the position of the one-way valve (12).

6. An atomiser according to claim 5, **characterised in that** the means for controlling the position of the one-way valve (12) includes: an opening in the first chamber (9) which is overlaid and closed by the one-way valve (12) such that a positive pressure in the first chamber (9) causes the one-way valve (12) to open.

7. An atomiser according to claim 6, **characterised in that** the first chamber (9) includes an air inlet, and the means for controlling the position of the one-way valve (12) further comprises an inlet one-way valve (10) which closes the inlet to the first chamber (9) unless a negative pressure is present in the first chamber (9).

8. An atomiser according to any one of claims 2 to 7, **characterised by** a rigid sidewall (14) separating the first and second chambers (9, 13).

9. An atomiser according to any one of claims 3 to 8, **characterised in that** the one-way valve (12) is made of a resilient material.

10. An atomiser according to any one of claims 6 to 9, **characterised in that** the area of the opening of the first chamber (9) closed by the one-way valve (12) is substantially greater than the area of the opening of the second chamber (13) closed by the one-way valve (12).

11. An atomiser according to claim 12, **characterised in that** the ratio of the area of the opening of the first chamber (9) to the area of the opening of the second chamber (13) is at least 10:1.

12. An atomiser according to any one of claims 2 to 11, **characterised in that** the secondary reservoir (1) includes a first entrance (25) leading to the second chamber (13), and a second entrance (26) leading into the secondary reservoir (1) from the reservoir (7), and **in that** the first entrance (25) is large relative to the second entrance (26).

13. An atomiser according to claim 12, **characterised in that** the first entrance (25) is at least 5mm across.

14. An atomiser according to claim 12 or 13, **characterised in that** the second entrance (26) is no larger than 1.4mm².

15. An atomiser according to claim 1, **characterised by** a one-way valve (12) which moves into an open position when the pressure within the atomiser is positive, and causes the means to prevent or stop atomisation to stop the passage of the product to the product outlet.

## Revendications

1. Atomiseur comprenant un gicleur d'atomisation, ce gicleur comportant un orifice de sortie de produit (5) à partir duquel le produit peut être atomisé, des moyens de source d'air comprimé, un réservoir de produit (7), des moyens (6, 8) de fourniture du produit provenant du réservoir (7), à l'orifice de sortie (5), une première chambre (9) dans laquelle le produit atomisé peut diffuser, et des moyens pour éviter ou stopper l'atomisation du produit,
**caractérisé en ce que**
les moyens pour éviter ou stopper l'atomisation du produit sont configurés pour permettre à une pression d'air positive à l'intérieur de l'atomiseur, d'être renvoyée dans les moyens (6, 8) de fourniture du produit entre le réservoir (7) et l'orifice de sortie de produit (5), de manière à stopper le passage du produit vers l'orifice de sortie de produit (5) en réponse à une pression positive dans l'atomiseur.

2. Atomiseur selon la revendication 1,
**caractérisé en ce que**
les moyens pour éviter ou stopper l'atomisation du produit comprennent un réservoir secondaire (1) à travers lequel le produit passe avant d'atteindre l'orifice de sortie de produit (5), et qui est en communication de fluide avec une seconde chambre (13), cette seconde chambre (13) pouvant être actionnée en réponse à une pression positive dans l'atomiseur, de manière à commander le fluide provenant du réservoir secondaire (1).

3. Atomiseur selon la revendication 2,
**caractérisé en ce que**
la seconde chambre (13) comprend une ouverture ; et l'atomiseur comprend en outre une soupape à un seul sens (12) qui ferme l'ouverture de la seconde chambre (13).

4. Atomiseur selon la revendication 3,
**caractérisé en ce que**
la soupape à un seul sens (12) est mobile entre une position fermant l'ouverture de la seconde chambre (13), cette position permettant au produit de passer du réservoir (7) à la sortie (5), et une position d'ouverture qui évite ou stoppe le passage du produit du réservoir (7) à l'orifice de sortie de produit (5).

5. Atomiseur selon la revendication 3 ou 4,
**caractérisé par**
des moyens pour commander la position de la soupape à un seul sens (12).

6. Atomiseur selon la revendication 5,
**caractérisé en ce que**
les moyens pour commander la position de la soupape à un seul sens (12) comprennent : une ouverture prévue dans la première chambre (9), cette ouverture étant recouverte et fermée par la soupape à un seul sens (12) de façon qu'une pression positive régnant dans la première chambre (9) produise l'ouverture de la soupape à un seul sens (12).

7. Atomiseur selon la revendication 6,
**caractérisé en ce que**
la première chambre (9) comprend une entrée d'air ; et les moyens pour commander la position de la soupape à un seul sens (12) comprennent en outre une soupape à un seul sens (10) qui ferme l'entrée de la première chambre (9) à moins qu'une pression négative soit présente dans la première chambre (9).

8. Atomiseur selon l'une quelconque des revendications 2 à 7,
**caractérisé par**
une paroi latérale rigide (14) séparant les première et seconde chambres (9, 13).

9. Atomiseur selon l'une quelconque des revendications 3 à 8,
**caractérisé en ce que**
la soupape à un seul sens (12) est réalisée dans un matériau élastique.

10. Atomiseur selon l'une quelconque des revendications 6 à 9,
**caractérisé en ce que**
la surface de l'ouverture de la première chambre (9) fermée par la soupape à un seul sens (12) est nettement supérieure à la surface de l'ouverture de la seconde chambre (13) fermée par la soupape à un seul sens (12).

11. Atomiseur selon la revendication 12,
**caractérisé en ce que**
le rapport de la surface de l'ouverture de la première chambre (9), à la surface de l'ouverture de la seconde chambre (13), est d'au moins 10 :1.

12. Atomiseur selon l'une quelconque des revendications 2 à 11,
**caractérisé en ce que**
le réservoir secondaire (1) comprend une première entrée (25) conduisant à la seconde chambre (13), et une seconde entrée (26) conduisant du réservoir (7) au réservoir secondaire (1), et
la première entrée (25) est grande par rapport à la seconde entrée (26).

13. Atomiseur selon la revendication 12,
**caractérisé en ce que**
la première entrée (25) est d'au moins 5 mm de large.

14. Atomiseur selon la revendication 11 ou 13,
**caractérisé en ce que**
la seconde entrée (26) ne dépasse pas 1,4 mm² de surface.

15. Atomiseur selon la revendication 1,
**caractérisé par**
une soupape à un seul sens (12) qui se déplace pour venir dans une position d'ouverture lorsque la pression régnant dans l'atomiseur est positive, et amène les moyens destinés à éviter ou stopper l'atomisation, à stopper le passage du produit vers l'orifice de sortie de produit.

## Patentansprüche

1. Zerstäuber mit einer Zerstäuberdüse, wobei die Zerstäuberdüse einen Produktauslass (5) aufweist, von dem aus das Produkt zerstäubt werden kann, Einrichtungen zum Beschaffen von Druckluft, einen Produktbehälter (7), Einrichtungen (6, 8) zum Befördern des Produkts vom Behälter (7) zum Auslass (5), eine erste Kammer (9), in die zerstäubtes Produkt diffundieren kann, und Einrichtungen zum Verhindern oder Stoppen, dass das Produkt zerstäubt wird,
**dadurch gekennzeichnet, dass**:
die Vorrichtung zum Verhindern oder Stoppen der Zerstäubung so aufgebaut ist, dass Überdruck im Zerstäuber zu der Vorrichtung (6, 8) zum Befördern des Produkts zwischen dem Behälter (7) und dem Produktauslass (5) zurückgeführt werden kann, um den Durchtritt des Produkts zum Produktauslass (5) im Ansprechen auf Überdruck im Zerstäuber zu stoppen.

2. Zerstäuber nach Anspruch 1, **dadurch gekennzeichnet, dass** die Vorrichtung zum Verhindern oder Stoppen der Zerstäubung einen Nebenbehälter (1) aufweist, durch den das Produkt hindurchtritt, bevor es den Produktauslass (5) erreicht, und der mit einer zweiten Kammer (13) in Flüssigkeitsverbindung steht, wobei die zweite Kammer (13) im Ansprechen auf Überdruck im Zerstäuber betrieben werden kann, um Flüssigkeit aus dem Nebenbehälter (1) zu treiben.

3. Zerstäuber nach Anspruch 2, **dadurch gekennzeichnet, dass** die zweite Kammer (13) eine Öffnung aufweist, und dass der Zerstäuber weiterhin ein Einwegeventil (12) aufweist, das die Öffnung in der zweiten Kammer (13) verschließt.

4. Zerstäuber nach Anspruch 3, **dadurch gekennzeichnet, dass** das Einwegeventil (12) zwischen einer Position bewegt werden kann, in der die Öffnung in der zweiten Kammer (13) verschlossen ist, was es dem Produkt ermöglicht, vom Behälter (7) zum Auslass (5) hindurchzutreten, und einer geöffneten Position, die verhindert oder stoppt, dass das Produkt vom Behälter (7) zum Produktauslass (5) hindurchtritt.

5. Zerstäuber nach Anspruch 3 oder 4, **gekennzeichnet durch** Einrichtungen zum Steuern der Position des Einwegeventils (12).

6. Zerstäuber nach Anspruch 5, **dadurch gekennzeichnet, dass** die Einrichtung zum Steuern der Position des Einwegeventils (12) aufweist: eine Öffnung in der ersten Kammer (9), die von dem Einwegeventil (12) so überlagert und verschlossen wird, dass Überdruck in der ersten Kammer (9) bewirkt, dass sich das Einwegeventil (12) öffnet.

7. Zerstäuber nach Anspruch 6, **dadurch gekennzeichnet, dass** die erste Kammer (9) einen Lufteinlass aufweist, und dass die Einrichtung zum Steuern der Position des Einwegeventils (12) weiterhin ein Einlass-Einwegeventil (10) aufweist, das den Einlass in die erste Kammer (9) verschließt, wenn sich in der ersten Kammer (9) kein Unterdruck befindet.

8. Zerstäuber nach einem der Ansprüche 2 bis 7, **gekennzeichnet durch** eine starre Seitenwand (14), die die ersten und zweiten Kammern (9, 13) voneinander trennt.

9. Zerstäuber nach einem der Ansprüche 3 bis 8, **dadurch gekennzeichnet, dass** das Einwegeventil (12) aus einem elastischen Material hergestellt ist.

10. Zerstäuber nach einem der Ansprüche 6 bis 9, **dadurch gekennzeichnet, dass** der durch das Einwegeventil (12) verschlossene Bereich der Öffnung der ersten Kammer (9) wesentlich größer ist, als der Bereich der durch das Einwegeventil (12) verschlossene Bereich der zweiten Kammer (13).

11. Zerstäuber nach Anspruch 12, **dadurch gekennzeichnet, dass** das Verhältnis des Bereichs der Öffnung der ersten Kammer (9) zum Bereich der Öffnung der zweiten Kammer (13) wenigstens 10:1 beträgt.

12. Zerstäuber nach einem der Ansprüche 2 bis 11, **dadurch gekennzeichnet, dass** der Nebenbehälter (1) einen ersten Eingang (25) aufweist, der in die zweite Kammer (13) führt, und einen zweiten Eingang (26), der vom Behälter (7) in den Nebenbehälter (1) führt, und dass der erste Eingang (25) im Verhältnis zum zweiten Eingang groß ist.

13. Zerstäuber nach Anspruch 12, **dadurch gekennzeichnet, dass** der erste Eingang (25) einen Durchmesser von wenigstens 5 mm aufweist.

14. Zerstäuber nach Anspruch 12 oder 13, **dadurch gekennzeichnet, dass** der zweite Eingang (26) nicht größer als 1,4 mm² ist.

15. Zerstäuber nach Anspruch 1, **gekennzeichnet durch** ein Einwegeventil (12), das sich in eine geöffnete Position bewegt, wenn im Zerstäuber Überdruck herrscht, und bewirkt, dass die Einrichtung zum Verhindern oder Stoppen der Zerstäubung den Durchtritt des Produkts zum Produktauslass stoppt.
